(19) Europäisches Patentamt / European Patent Office / Office européen des brevets

(11) **EP 2 173 349 B1**

## (12) FASCICULE DE BREVET EUROPEEN

(45) Date de publication et mention de la délivrance du brevet:
**09.03.2011 Bulletin 2011/10**

(21) Numéro de dépôt: **08827826.2**

(22) Date de dépôt: **02.07.2008**

(51) Int Cl.:
**A61K 31/4745** [(2006.01)]     **A61K 38/16** [(2006.01)]
**A61P 35/00** [(2006.01)]

(86) Numéro de dépôt international:
**PCT/FR2008/000943**

(87) Numéro de publication internationale:
**WO 2009/024667 (26.02.2009 Gazette 2009/09)**

(54) **COMBINAISONS ANTITUMORALES CONTENANT UN AGENT INHIBITEUR DE VEGF ET DE L'IRINOTECAN**

ANTITUMOR-KOMBINATIONEN MIT EINEM VEGF-HEMMENDEN MITTEL UND IRINOTECAN

ANTITUMOUR COMBINATIONS CONTAINING A VEGF INHIBITING AGENT AND IRINOTECAN

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **05.07.2007 FR 0704868**

(43) Date de publication de la demande:
**14.04.2010 Bulletin 2010/15**

(73) Titulaire: **Aventis Pharma S.A.**
**92160 Antony (FR)**

(72) Inventeurs:
• **BISSERY, Marie-Christine**
  **F-75013 Paris (FR)**
• **CHIRON-BLONDEL, Marielle**
  **F-75013 Paris (FR)**
• **LEJEUNE, Pascale**
  **F-75013 Paris (FR)**
• **VRIGNAUD, Patricia**
  **F-75013 Paris (FR)**

(74) Mandataire: **Le Pennec, Magali et al**
**Sanofi-Aventis**
**Direction Brevets**
**174 Avenue de France**
**75013 Paris (FR)**

(56) Documents cités:
**WO-A-2005/011734**

• VANHOEFER U ET AL: "IRINOTECAN IN COMBINATION WITH NEW AGENTS" EUROPEAN JOURNAL OF CANCER. SUPPLEMENT, PERGAMON, OXFORD, GB, vol. 2, no. 7, juin 2004 (2004-06), pages 14-20, XP001207039 ISSN: 1359-6349
• HOFF PAULO M ET AL: "A phase I study of escalating doses of the tyrosine kinase inhibitor semaxanib (SU5416) in combination with irinotecan in patients with advanced colorectal carcinoma" JAPANESE JOURNAL OF CLINICAL ONCOLOGY, vol. 36, no. 2, février 2006 (2006-02), pages 100-103, XP008087904 ISSN: 0368-2811 (print) 1465-3621(ele
• JONES-BOLIN, SUSAN ET AL: "The effects of the oral, pan- VEGF -R kinase inhibitor CEP-7055 and chemotherapy in orthotopic models of glioblastoma and colon carcinoma in mice" MOLECULAR CANCER THERAPEUTICS , 5(7), 1744-1753 CODEN: MCTOCF; ISSN: 1535-7163, 2006, XP002466373
• YOKOI KENJI ET AL: "Dual inhibition of epidermal growth factor receptor and vascular endothelial growth factor receptor phosphorylation by AEE788 reduces growth and metastasis of human colon carcinoma in an orthotopic nude mouse model" CANCER RESEARCH, vol. 65, no. 9, mai 2005 (2005-05), pages 3716-3725,3707, XP002466374 ISSN: 0008-5472
• YAZICI YASEMIN D ET AL: "Antivascular therapy of oral tongue squamous cell carcinoma with PTK787." THE LARYNGOSCOPE DEC 2005, vol. 115, no. 12, décembre 2005 (2005-12), pages 2249-2255, XP008087914 ISSN: 0023-852X

EP 2 173 349 B1

**Description**

La présente invention concerne les combinaisons d'un inhibiteur de VEGF, le VEFG Trap, et d'un agent chimiotoxique de classe des inhibiteurs de topoisomerase utile dans le traitement des maladies néoptastiques, l'irinotecan.

Les inhibiteurs de VEGF qui sont des inhibiteurs du facteur de croissance vasculaire endothélial sont dans la majorité des cas des produits biologiques choisis parmi les récepteurs solubles, les antisens, les aptamères de RNA et les anticorps. Les inhibiteurs de topoisomérase utiles dans le traitement des maladies néoplastiques connues sont choisis parmi les camptothécines, dont le CPT 11, le topotecan et le pyridobenzoindole. La présente combinaison vise en particulier le traitement du cancer du colon ou de l'estomac.

La description et la préparation de l'inhibiteur du VEGF utilisé dans l'invention est une protéine chimère VEGF-Trap et est décrite dans la demande de brevet WO00/75319. Il y a plusieurs formes de réalisation de la protéine chimère.

La forme de réalisation correspondant au VEGF-Trap est celle décrite à la figure 24 (séquence). Le VEGF Trap utilisé dans l'invention est une protéine de fusion comprenant la séquence signal du VEGFR1 fusionnée au domaine Ig D2 du récepteur VEGFR1, elle-même fusionnée au domaine Ig D3 du récepteur VEGFR2 fusionnée à son tour au domaine Fc de IgG1 encore appelée VEGFR1 R2-FcAC1 ou le Flt1D2.Flk1D3.FcΔC1.

Généralement les doses de VEGF Trap utilisées chez l'homme , qui dépendent des facteurs propres au sujet à traiter, sont comprises entre 1 et 10 milligrammes par kilo quand l'administration est faite par voie sous cutanée ou par voie intraveineuse.

Parmi les inhibiteurs de toposiomerase on utilise l'irinotecan encore connu sous la dénomination commune de CPT-11.

L'irinotecan est généralement utilisé par voie intraveineuse à une dose comprise entre 100 mg/m$^2$ et 500 mg/m$^2$ selon le schéma d'administration. On utilise par exemple une dose de 150 mg/m$^2$ pour un schéma hebdomadaire et une dose comprise entre 200 et 400 mg/m$^2$ pour un schéma toutes les trois semaines..

Il a été décrit dans un article de H Hurwitz, L Fehrenbacher, W Novotny, T Cartwright, J Hainsworth, W Heim, J Berlin, A Baron, S Griffing, E Holmgren, N Ferrera, G Fyfe, B Rogers, R Ross, F Kabbinavar paru dans « The New England Journal of Medecine » un essai clinique prouvant un meilleur taux de survie lors de l'utilisation de la combinaison de bevacizumab avec l'irinotecan, le 5FU et la leucoveurine par rapport à la même combinaison ne contenant pas le bevacizumab. Rien ne prouve dans cet essai clinique que l'amélioration du taux de survie provient de la combinaison de l'irinotecan avec le bevacizumab, elle peut aussi bien provenir de la combinaison du 5FU ou de la leucovorine avec le bevacizumab ou peut provenir de la quatruple combinaison. Or comme il est connu que chacun des agents anticancéreux apporte à côté de son effet thérapeutique des effets secondaires toxiques il semble opportun de limiter au maximum leur présence surtout quand le même effet peut être obtenu en l'absence d'au moins l'un d'entre eux. D'autre part cet article ne prouve aucun effet synergique au sens de Corbett c'est à dire un effet qui ne peut pas être obtenu avec chacun des élements de la combinaison utilisé seul à sa dose maximale tolérée.

Le VEGF Trap est un récepteur soluble crée par la fusion du deuxième domaine Ig du VEGFR-1 avec le troisième domaine Ig du VEGFR-2 ensuite fusionné à la partie Fc d'une IgG1 humaine. Comme le récepteur VEGFR-1, l'aflibercept (VEGF Trap) a une très haute affinité pour le VEGF-A avec un Kd de 0.5 picoM. La liaison de haute affinité du VEGF Trap avec le VEGF-A aboutit à la formation d'un complexe qui empêche le VEGF de se fixer et d'activer ses récepteurs à la surface des cellules.

En comparaison avec l'Avastin (ou bevacizumab) le VEGF Trap est un récepteur soluble alors que l'Avastin est un anticorps dirigé contre le VEGF-A. Le VEGF Trap a une affinité pour le VEGF-A très supérieure à celle de l'Avastin et un profil de sélectivité différent car le VEGF Trap se lie également aux autres ligands des récepteurs VEGFR1-2, c'est à dire au PIGF (placental growth factor) et au VEGF-B. D'autres parts, le VEGF Trap a un poids moléculaire sensiblement inférieur à celui de l'Avastin (115 kDa pour l'aflibercept versus 160 kDa pour l'Avastin) plus favorable à la pénétration dans les tumeurs solides.

Il a maintenant été trouvé, et c'est ce qui fait l'objet de la présente invention, que l'efficacité du VEGF Trap peut être considérablement améliorée lorsqu'il est administré en association avec une substance thérapeutiquement utile dans les traitements anticancéreux ayant un mécanisme d'action différent de celui des inhiteurs du VEGF, l'irinotecan

Par ailleurs, l'activité des produits dépendant des doses utilisées, il est possible d'utiliser des doses plus élevées et d'augmenter l'activité en diminuant les phénomènes de toxicité ou en retardant leur apparition en associant à inhibiteurs de VEGF d'autres substances thérapeutiquement actives des facteurs de croissance de type hématopoïétique tels que le G-CSF ou le GM-CSF ou certaines interleukines.

l'invention concerne les associations de VEGF Trap avec l'irinotecan.

L'efficacité améliorée d'une combinaison selon l'invention peut être mise en évidence par la détermination du synergisme thérapeutique.

Une combinaison manifeste un synergisme thérapeutique si elle est thérapeutiquement supérieure à l'un et à l'autre des constituants utilisé à sa dose optimale.

Pour mettre en évidence l'efficacité d'une combinaison, il peut être nécessaire de comparer la dose maximale tolérée de la combinaison à la dose maximale tolérée de chacun des constituants isolés dans l'étude considérée. Cette efficacité peut être quantifiée, par exemple par le $\log_{10}$ des cellules tuées qui est déterminé selon la formule suivante :

$$\log_{10} \text{ des cellules tuées} = \text{T-C (jours)}/3.32 \times T_d$$

dans laquelle T - C représente le délai de croissance des cellules qui est le temps moyen, en jours, pour que les tumeurs du groupe traité (T) et les tumeurs du groupe témoin (C) aient atteint une valeur prédéterminée (1 g par exemple) et $T_d$ représente le temps, en jours, nécessaire au doublement du volume de la tumeur chez les animaux témoins [T.H. CORBETT et coll., Cancer, 40, 2660.2680 (1977) ; F.M. SCHABEL et coll., Cancer Drug Development, Part B, Methods in Cancer Research, 17, 3-51, New-York, Academic Press Inc. (1979)] Un produit est considéré comme actif si $\log_{10}$ des cellules tuées est supérieur ou égal à 0,7. Un produit est considéré comme très actif si le $\log_{10}$ des cellules tuées est supérieur à 2,8.

La combinaison, utilisée à sa dose maximale tolérée propre, dans laquelle chacun des constituants sera présent à une dose généralement inférieure ou égale à sa dose maximale tolérée, manifestera une synergie thérapeutique lorsque le $\log_{10}$ des cellules tuées sera supérieur à la valeur du $\log_{10}$ des cellules tuées du meilleur constituant lorsqu'il est administré seul, et notamment d'une supériorité d'au moins un log cell kill.

L'efficacité des combinaisons sur les tumeurs solides peut être déterminée expérimentalement de la manière suivante:

Les animaux soumis à l'expérience, généralement des souris, sont greffés bilatéralement par voie sous-cutanée avec 30 à 60 mg d'un fragment de tumeur humaine HCT116 (BRATTAIN, M.G., FINE, W.D., KHALED, F.M., THOMPSON, J. and BRATTAIN, D.E., Heterogeneity of malignant cells from a human colonic carcinoma. Cancer Res.,1981, 41, 1751-1756.) au jour 0. Les animaux portant les tumeurs sont randomisés avant d'être soumis aux divers traitements et contrôles. Dans le cas de traitement de tumeurs de la présente invention, on a laissé les tumeurs se développer jusqu'à une taille comprise entre 48 et 294 mg, ce qui a permis d'avoir une tumeur médiane par groupe comprise entre 129 et 162 mg, Les animaux qui ont subi le traitement avec le VEGF Trap seul présentaient un poids compris entre 17.1 et 22.7 g, les animaux ayant subi le traitement avec l'irinotecan seul avaient un poids compris entre 17.5 et 22.3 g et ceux qui ont reçu la combinaison avaient un poids compris entre 17.5 et 23.6 g. Des animaux porteurs de tumeurs ont également été soumis aux mêmes traitements avec l'excipient seul afin de pouvoir dissocier l'effet toxique de l'excipient de l'effet propre de la chimiothérapie sur la tumeur. La chimiothérapie a commencé le 12 ème jour après la greffe tumorale. Les injections de VEGF Trap ont été faites par voie sous cutanée simultanément aux injections d'irinotécan qui elles ont été faites par voie intraveineuse selon une double injection quotidienne. Ces injections ont été réalisées aux jours 12, 15 et 18 après l'implantation de la tumeur. Les différents groupes d'animaux sont pesés trois ou quatre fois par semaine jusqu'à ce que la perte maximale de poids soit atteinte puis les groupes sont pesés au moins une fois par semaine jusqu'à la fin de l'essai.

Les tumeurs sont mesurées deux ou trois fois par semaine jusqu'à ce que la tumeur atteigne environ 2 g ou jusqu'à la mort de l'animal si celle-ci survient avant que la tumeur atteigne 2 g. Les animaux sont autopsiés lors du sacrifice.

L'activité antitumorale est déterminée en fonction des différents paramètres enregistrés.

A titre d'exemples, sont donnés, dans les tableaux suivants les résultats obtenus avec des combinaisons du VEGF Trap et de l'irinotecan utilisées à leur dose optimale.

La présente invention concerne également les kits de compositions pharmaceutiques contenant les produits utilisées dans les combinaisons selon l'invention.

Les produits qui constituent la combinaison peuvent être administrés simultanément, séparément ou d'une manière étalée dans le temps de façon à obtenir le maximum d'efficacité de la combinaison ; chaque administration pouvant avoir une durée variable allant d'une administration totale rapide à une perfusion continue.

Il en résulte que, au sens de la présente invention, les combinaisons ne sont pas uniquement limitées à celles qui sont obtenues par association physique des constituants, mais aussi à celles qui permettent une administration séparée qui peut être simultanée ou étalée dans le temps.

Les compositions selon l'invention sont de préférence les compositions administrables par voie parentérale.

Les compositions pour administration parentérale sont généralement des solutions ou des suspensions stériles pharmaceutiquement acceptables qui peuvent éventuellement être préparées extemporanément au moment de l'emploi. Pour la préparation de solutions ou de suspensions non aqueuses peuvent être utilisées des huiles végétales naturelles telles que l'huile d'olive, l'huile de sésame ou l'huile de paraffine ou les esters organiques injectables tels que l'oléate d'éthyle. Les solutions stériles aqueuses peuvent être constituées d'une solution du produit dans l'eau. Les solutions aqueuses conviennent pour l'administration intraveineuse dans la mesure où le pH est convenablement ajusté et où l'isotonicité est réalisée, par exemple par une quantité suffisante de chlorure de sodium ou de glucose. La stérilisation peut être réalisée par chauffage ou par tout autre moyen qui n'altère pas la composition. Les combinaisons peuvent aussi se présenter sous forme de liposomes ou sous forme d'association avec des supports tels que les cyclodextrines ou les polyéthylèneglycols.

Dans les combinaisons selon l'invention dont l'application des constituants peut être simultanée, séparée ou étalée dans le temps, il est particulièrement avantageux que la quantité de dérivé du VEGF Trap représente de 10% à 80% en poids de la combinaison cette teneur pouvant varier en fonction de la nature de la substance associée, de l'efficacité recherchée et de la nature du cancer à traiter.

Les combinaisons selon l'invention sont particulièrement utiles dans le traitement des cancers du colon et/ou de l'estomac. En particulier, elles peuvent présenter l'avantage de pouvoir mettre en oeuvre les constituants à des doses nettement plus faibles que celles auxquelles ils sont utlilisés seuls.

L'exemple suivant illustre une combinaison selon l'invention.

EXEMPLE

On prépare selon la technique habituelle, pour l'administration sous cutanée, des ampoules de 1 cm3 contenant 25 mg de VEGF Trap qui sont diluées dans un tampon phosphate 5mM, citrate de sodium 5 mM, chlorure de sodium 100 mM, polysorbate 20 et sucrose 20%. Le volume d'administration par souris est 0.1 ml. Le VEGF Trap est administré une fois par jour, aux jours 12, 15 et 18 apres l'implantation de la tumeur.

On prépare selon la technique habituelle, pour l'administration intraveineuse 0,3 ml par souris à partir d'une solution commerciale à 20 mg/ml d'irinotecan à diluer avec du dextrose 5 % dans l'eau.

Ces solutions sont administrées simultanément, après dilution convenable,

Le traitement avec l'irinotécan est répété deux fois par jour à 4 heures d'intervalle, aux jours 12, 15 et 18, apres l'implantation de la tumeur

Les résultats de l'essai sont joints dans le tableau annexé.

Temps de doublement de la tumeur = 3.2 jours.

Abbreviations utilisées: (T-C) délai de croissance de la tumeur, Ick = log de cellules tuées.

Il a été observé une toxicité pour l'irinotecan seul aux doses de 52.4, 32.5 et 20.2 mg/kg/injection due à une mort au dosage de 52.4 et une perte de poids supérieure à 20% pour les deux dosages inférieurs. Ainsi la dose maximale tolérée pour l'irinotecan a été de 12.5 mg/kg/inj (dose totale injectée de 75.0 mg/kg). La dose de 12.5 mg/kg/injection a été trouvée active avec un Ick de 1.8.

Pour le VEGF Trap le produit a été bien toléré à tous les dosages testés et a été trouvé actif avec un Ick de 1.7 à 40 mg/kg/administration et 25 mg/kg/administration. La dose inférieure de 10 mg/kg/administration est également active avec un Ick de 1.3. La dose de 2.5mg/kg/administration est inactive.

Pour la combinaison d'irinotecan à 32.5 mg/kg/inj quelle que soit la dose de VEGF Trap, la combinaison a été trouvée toxique avec une perte de poids de 18% proche de la toxicité. La dose inférieure de 20.2 mg/kg/inj d'irinotecan avec 40 mg/kg de VEGF Trap a été considérée comme la dose maximale tolérée. Cette dose a présenté un Ick de 3.0 jugée très active. Le même niveau d'activité a été trouvé avec les doses inférieures de VEGF Trap comme 25, 10, 2,5 mg/kg/administration (Ick de 2.9, 3.0 et 2.9 respectivement).

L'irinotecan à 12.5 mg/kg/inj associé au VEGF Trap à 40 mg/kg/administration est actif avec un Ick de 2.7. Cette activité anti-tumorale est maintenue avec 25 et 10 mg/kg de VEGF Trap (Ick de 2.9 et 2.7, respectivement). La combinaison avec 2.5 mg/kg/administration de VEGF Trap a une activité de 2.0 Ick.

En conclusion l'activité de la combinaison de VEGF Trap avec l'irinotecan montre un effet synergistique avec un log cell kill de 3.0, à la dose maximale tolérée de la combinaison, qui correspond à plus de 1 log cell kill comparée à l'activité de chacun des composés utilisé seul qui présente un log cell kill de 1.8 et 1.7.(pour l' irinotecan à 12.5 mg/kg/ injection et VEGF Trap à 40 mg/kg/administration, respectivement). Une activité antitumorale est maintenue à plusieurs niveaux de doses en dessous de la dose maximale tolérée de la combinaison.

| Groupe | Agent seul) | Voie d'adm | Dosage en mg/kg par injection | Schema en jours | dose totale in mg/kg | Mort à cause du produit avec jour de la mort | Perte en poids de la souris (jour du nadir) | Temps moyen pour la tumeur pour atteindre 750mg en jours | T-C in day | log cell kill | Survivants sans tumeur au jour 144 | Comments |
|---|---|---|---|---|---|---|---|---|---|---|---|---|
| 6 | CPT-11 | i.v. 0.3ml | 52.4 | 12, 15, 18 | 314.4 | 1/8 (23) | -27.0 (21) | - | - | - | - | Toxic |
| 7 | | | 32.5 | (2x/d) | 195.0 | 0/7 | -23.1 (19) | - | - | - | - | Toxic |
| 8 | | | 20.2 | (4h apart) | 121.2 | 0/8 | -21.9 (19) | - | - | - | - | Toxic |
| 9 | | | 12.5 | | 75.0 | 0/8 | -15.9 (21) | 37.6 | 19.1 | 1.8 | 0/8 | HNTD active |
| | | | | | | | | | | | | |
| 2 | VEGF Trap | s.c. 0.1ml | 40.0 | 12,15,18 | 120.0 | 0/8 | -3.1 (13) | 36.8 | 18.3 | 1.7 | 0/8 | HDT active |
| 3 | | | 25.0 | | 75.0 | 0/8 | -2.2 (13) | 36.8 | 18.3 | 1.7 | 0/8 | active |
| 4 | | | 10.0 | | 30.0 | 0/8 | -5.0 (19) | 32.0 | 18.3 | 1.3 | 1/8 | active |
| 5 | | | 2.5 | | 7.5 | | -8.3 (19) | 20.2 | 1.7 | 0.2 | 018 | inactive |

| | | | | | | | | | | | | |
|---|---|---|---|---|---|---|---|---|---|---|---|---|
| 10 | CPT-11 VEGF Trap | i.v 0.3ml s.c. 0.1ml | 32.5 40.0 | 12,15,18 (2x/j) 12,15,18 | 195.0 120.0 | 0/8 [b] | - 16.2 (21) | - | - | - | - | Toxic |
| 11 | | | 32.5 25.0 | | 195.0 75.0 | 0/8 | - 18.6 (21) | - | - | - | - | Toxic |
| 12 | | | 32.5 10.0 | | 195.0 30.0 | 0/8 | - 18.2 (19) | - | - | - | - | Toxic |
| 13 | | | 32.5 2.5 | | 195.0 7.5 | 0/8 | - 18.4 (19) | - | - | - | - | Toxic |
| 14 | | | 20.2 40.0 | | 121.2 120.0 | 0/8 | - 13.0 (21) | 50.9 | 32.4 | 3.0 | 0/8 | HNTD hautement active |
| 15 | | | 20.2 25.0 | | 121.2 75.0 | 0/8 | - 11.9 (19) | 49.6 | 31.1 | 2.9 | 0/8 | haut active |
| 16 | | | 20.2 10.0 | | 121.2 30.0 | 0/8 | - 13.0 (19) | 50.7 | 32.2 | 3.0 | 0/8 | haut active |
| 17 | | | 20.2 2.5 | | 121.2 7.5 | 0/8 | - 12.5 (19) | 49.7 | 31.2 | 2.9 | 0/8 | haut active |
| 18 | | | 12.5 40.0 | | 75.0 120.0 | 0/8 | - 11.0 (19) | 46.8 | 28.3 | 2.7 | 0/8 | active |
| 19 | | | 12.5 25.0 | | 75.0 75.0 | 0/8 | - 11.9 (19) | 49.8 | 31.3 | 2.9 | 0/8 | haut active |
| 20 | | | 12.5 10.0 | | 75.0 30.0 | 0/8 | - 10.0 (21) | 47.0 | 28.5 | 2.7 | 0/8 | active |
| 21 | | | 12.5 2.5 | | 75.0 7.5 | 0/8 | - 14.3 (21) | 40.0 | 21.5 | 2.0 | 0/8 | active |

(suite)

| | | | | | | | |
|---|---|---|---|---|---|---|---|
| 1 | Vehicle | s.c. 0.1 ml | - | 12, 15, 18 | -9.6 (25) | 18.5 | 0/10 |
| | | i.v. 0.3ml | - | 12,15,18 (2x/j) (4h après) | | | |

**Revendications**

1.  Combinaisons contenant le VEGF Trap avec de l'irinotecan thérapeutiquement utiles dans le traitement des maladies néoplastiques.

2.  Combinaisons selon la revendication 1 **caractérisées en ce qu'**elles contiennent de 10% à 80 % en poids de VEGF Trap.

3.  Produits contenant le VEGF Trap et de l'irinotecan dans le traitement des maladies néoplasiques comme préparation combinée pour une utilisation simultanée, séparée ou étalée dans le temps en thérapie anticancéreuse.

4.  Combinaisons contenant le VEGF Trap avec de l'irinotecan à l'exclusion de tout autre dérivé chimiotoxique présentant un effet thérapeutiquement synergique dans le traitement des maladies néoplasiques.

**Claims**

1.  Combinations containing VEGF Trap with irinotecan, of therapeutic use in the treatment of neoplastic diseases.

2.  Combinations according to Claim 1, **characterized in that** they contain from 10% to 80% by weight of VEGF Trap.

3.  Products containing VEGF Trap and irinotecan in the treatment of neoplastic diseases, as a combined preparation for simultaneous, separate or sequential use in anticancer therapy.

4.  Combinations containing VEGF Trap with irinotecan with the exclusion of any other chemotoxic derivative having a therapeutically synergistic effect in the treatment of neoplastic diseases.

**Patentansprüche**

1.  Kombinationen, enthaltend VEGF Trap zusammen mit Irinotecan, die bei der Behandlung von neoplastischen Krankheiten therapeutisch nützlich sind.

2.  Kombinationen nach Anspruch 1, **dadurch gekennzeichnet, daß** sie 10 bis 80 Gew.-% VEGF Trap enthalten.

3.  Produkte enthaltend VEGF Trap sowie Irinotecan bei der Behandlung von neoplastischen Krankheiten als Kombinationspräparat für die gleichzeitige, getrennte oder zeitlich gestaffelte Verwendung bei der Antikrebstherapie.

4.  Kombinationen enthaltend VEGF Trap gemeinsam mit Irinotecan unter Ausschluß jeglichen sonstigen chemotoxischen Derivats mit therapeutisch synergistischer Wirkung bei der Behandlung von neoplastischen Krankheiten.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• WO 0075319 A

**Littérature non-brevet citée dans la description**

• **H Hurwitz ; L Fehrenbacher ; W Novotny ; T Cartwright ; J Hainsworth ; W Heim ; J Berlin ; A Baron ; S Griffing ; E Holmgren.** *The New England Journal of Medecine*
• **T.H. CORBETT.** *Cancer,* 1977, vol. 40, 2660-2680
• Cancer Drug Development, Part B. **F.M. SCHABEL.** Methods in Cancer Research. Academic Press Inc, 1979, vol. 17, 3-51
• **BRATTAIN, M.G. ; FINE, W.D. ; KHALED, F.M. ; THOMPSON, J. ; BRATTAIN, D.E.** Heterogeneity of malignant cells from a human colonic carcinoma. *Cancer Res.,* 1981, vol. 41, 1751-1756